# EUROPEAN PATENT SPECIFICATION

(11) **EP 2 043 663 B1**
(45) Date of publication and mention of the grant of the patent: **19.03.2014**
(21) Application number: 07748333.7
(22) Date of filing: 16.07.2007
(51) Int. Cl.: A61K 33/04, A61K 33/06, A61K 33/30, A61K 9/08, A61K 9/107, A61K 9/70, A61P 17/12

(54) **TOPICAL TREATMENT OF WARTS**
TOPISCHE WARZENBEHANDLUNG
TRAITEMENT TOPIQUE DE VERRUES

(30) Priority: 25.07.2006 SE 0601605
(43) Date of publication of application: 08.04.2009
(73) Proprietor: ITH Immune Therapy Holdings AB, 171 76 Stockholm (SE)
(72) Inventor: LARSSON, Patrik, S-754 40 Uppsala (SE); WINQVIST, Ola, S-756 53 Uppsala (SE)
(74) Representative: Boult Wade Tennant
(86) International application number: PCT/SE2007/000691
(87) International publication number: WO 2008/013493

(56) References cited:
- WO-A2-2005/069735
- GB-A- 2 165 453
- US-A- 3 683 076
- US-A- 3 985 674
- US-A- 4 826 677
- US-A- 4 900 538
- US-A- 5 391 546
- US-A1- 2003 235 627
- US-A1- 2005 123 625

## Description

### FIELD OF THE INVENTION

The present invention relates to a means for use in a method for topical treatment of warts, in particular common warts (*Verruca vulgaris*).

### BACKGROUND OF THE INVENTION

Common and other warts are caused by human papilloma virus (HPV) infection of the epithelium; a great number of HPV varieties capable of giving rise to warts are known. Warts form slowly growing coarse protuberances, which are often circular in form and fair or grey in colour. Plantar or mosaic and other forms of warts mainly occur on the soles of the feet, in particular on their weight bearing portions. Except for genital warts from which carcinomas may form epithelial infection by HPV is generally benign but often irritating and sometimes painful as well as disfigurating.

Warts are treated topically with keratolytic agents such as salicylic or lactic acid, which have to be applied on the warts for a longer period of time such as for one week or more. Other treatments comprise the use of toxic antiviral agents dispensed only by prescription and which, like podophylline, may irritate the skin. Warts may also removed by repeated cryotherapy and by surgery, in particular by laser techniques. Cryotherapy and surgery is carried out by physicians or specially trained nurses, and thus is costly. Instruments for self-treatment by mechanical abrasion of warts in combination with the topical application of a pharmacologically active agent like pyruvic acid are known from, i.a. U.S. patent 6,585,742 (Stough). Their use by a patient may however result in an infection of lesions produced during abrasion.

U.S. Patent No. 6,821,523 discloses the treatment of warts by topical application of a composition having a pH of from 8.0 to 13.0 comprising inorganic hydroxide or a nitrogenous base in a pharmaceutically acceptable topical carrier, the composition being in the form of a bioadhesive, in a plaster or in a skin patch. The known composition is applied warts twice daily over a period of several weeks. After two weeks' treatment most warts were found to have been significantly reduced in size whereas, after treatment for eight weeks, most had entirely regressed.

In spite of the various methods for treating common and other warts known in the art there is room for improvement, in particular in regard of the length, complexity and/or cost of treatment.

Dithionites (hyposulphites), like sodium and zinc dithionite, are salts of the unstable dithionic (hyposulphuric) acid H₂S₂O₄. They are strong reducing agents used for bleaching of pulp and materials containing iron(III)compounds, animal hair, straw, viscose and nylon. Dithionites and their aqueous solutions are sensitive to air. They are prone to react vigorously with small amounts of water.

WO 2003/020036 discloses fungicidal compositions comprising dithionite. CS 242641 discloses an agent for skin cleaning and protection comprising sodium dithionite. PL 19640805 discloses a soap useful for removing stains from skin.

US Patent 4,826,677 discloses liquid preparations of film-forming polymers for the topical treatment of psoriasis. These liquid preparations include anti-psoriatic ingredients in particular, dithranol and/or glucocorticoid in combination with keratolytically acting urea.

US Patent 3,683,076 discloses injectable aqueous solutions for the treatment of osteoarthritis. These solutions include glucosamine sulphate and glucosamine hydroiodide stabilised with sodium hypersulphate.

Patent Application No. GB 2,165,453 discloses glucocorticoid-free pharmaceutical agents for the treatment of psoriasis, comprising a self-adhesive non-permeable film containing an anti-psoriatic active substance in a uniform fine distribution.

### OBJECTS OF THE INVENTION

It is an object of the present invention to provide a method of topically treating warts, in particular common warts, that that lacks at least some of the drawbacks of the methods known in the art.

It is another object of the invention to provide a means for use in the method.

Further objects of the invention will become apparent from the following summary of the invention, preferred embodiments thereof, and the appended claims.

### SUMMARY OF THE INVENTION

The present invention provides a pharmaceutical composition for use in the treatment of common warts (Verruca vulgaris) wherein the composition is for topical administration and comprises any of sodium, potassium, magnesium, calcium and zinc dithionite and their mixtures in a pharmaceutically acceptable aqueous carrier selected from water or a mixture of water and non-reducible organic solvent selected from ethanol, ethylene glycol, glycerol, and their mixtures, and wherein the dithionite from which the aqueous solution has been prepared is stabilized by any of sodium hydroxide, potassium hydroxide, sodium carbonate, potassium carbonate, magnesium hydroxide, magnesium oxide, magnesium carbonate, basic magnesium carbonate, calcium hydroxide, calcium oxide, basic aluminium oxide, zinc oxide, trisodium phosphate, sodium and potassium salts of organic acids, such as sodium acetate, disodium maleate and trisodium citrate, and their mixtures, with the proviso that the pharmaceutical composition does not comprise an organo-silicon compound.

In the context of the present invention the purpose of "treating warts/treatment of warts" is to make them removable by hand, that is, to sufficiently soften them for easy removal. The softening results in a wart to be transformed to a soft or spongy mass.

The present invention does not contemplate the use of an organo-silicon compound.

According to the present invention is disclosed a means for use in a method of treating warts, in particular common warts (*Verruca vulgaris*), comprising topical administration of a dithionite in a pharmaceutically acceptable carrier to a wart, keeping the dithionite in the carrier in contact with the wart for a selected period of time so as substantially soften the wart, optionally repeating administration and contact, and mechanically removing the wart. The method does not leave any scars on the skin from which the wart has been removed. Neither does it irritate the skin.

Preferred dithionites for use according to the invention are sodium, potassium, magnesium, calcium and zinc dithionite. A preferred carrier is water or mixtures of water and non-reducible organic solvents such as ethanol, ethylene glycol and glycerol. In the context of the present invention "non-reducible" refers to reduction by dithionite.

According to a first preferred aspect of the invention the composition comprises a non-reducible gelling agent. The non-reducible gelling agent is preferably selected from gel forming cellulose derivatives such as hydroxypropyl cellulose, (HPC) and hydroxymethylpropyl cellulose (HPMC), cross-linked polyvinylpyrrolidone, and cross-linked polyvinyl alcohol.

According to another aspect the carrier is a water-in-oil or, preferably, an oil-in-water emulsion, comprising a non-reducible oil such as lanolin or cetyl alcohol and a non-reducible dispersant such as polyethylene glycol.

According to a further preferred aspect of the invention the crystalline dithionite, from which the aqueous solution of the invention is prepared, is stabilized, in particular by sodium hydroxide, potassium hydroxide, sodium carbonate, sodium bicarbonate, potassium carbonate, potassium bicarbonate magnesium hydroxide, magnesium oxide, magnesium carbonate, basic magnesium carbonate, calcium hydroxide, calcium oxide, basic aluminium oxide, zinc oxide, trisodium phosphate, sodium and potassium salts of organic acids, such as sodium acetate, disodium maleate and trisodium citrate. Other useful stabilizing agents known in the art are formaldehyde and glycerol. Stabilized aqueous solutions of dithionite are known in the art (U.S. Patent No. 3,985,674 (Ellis et al.). They may be used in the present invention in form of ex tempore preparations since their useful shelf life at room temperature is too short to allow marketing as a pharmaceutical.

According to the present invention is also disclosed a pharmaceutical composition for topical treatment of warts, in particular common warts (Verruca vulgaris), comprising a pharmacologically effective amount of dithionite dissolved in a pharmaceutically acceptable aqueous carrier, wherein the dithionite is selected from sodium, potassium, magnesium, calcium and zinc dithionite, and their mixtures, and wherein the carriers are water and mixtures of water and non-reducible organic solvent such as ethanol, ethylene glycol, glycerol, and their mixtures.

According to a further preferred aspect the composition of the invention comprises a non-reducible gelling agent, which preferably consists or comprises a cellulose derivative or a mixture of cellulose derivatives. Further useful gelling agents include cross-linked polyvinylpyrrolidone and cross-linked polyvinyl alcohol.

According to a further aspect the carrier is comprised by an oil-in-water emulsion.

According to a further preferred aspect of the invention the dithionite from which the aqueous solution is prepared is stabilized, in particularly by any of sodium hydroxide, potassium hydroxide, sodium carbonate, potassium carbonate, magnesium hydroxide, magnesium oxide, magnesium carbonate, basic magnesium carbonate, calcium hydroxide, calcium oxide, basic aluminium oxide, zinc oxide, trisodium phosphate, sodium and potassium salts of organic acids, such as sodium acetate, disodium maleate and trisodium citrate, and their mixtures.

Additionally disclosed is the use of the composition of the invention for topical treatment of warts.

According to a further preferred aspect of the invention is disclosed a medical patch comprising solid dithionite in a non-reducible matrix for application to warts upon wetting the patch by an aqueous medium. The solid dithionite in the patch is selected from sodium, potassium, magnesium, calcium and zinc dithionite, and their mixtures, and is optionally stabilized by, for instance, any of sodium hydroxide, potassium hydroxide sodium carbonate, potassium carbonate, magnesium hydroxide, magnesium oxide, magnesium carbonate, basic magnesium carbonate, calcium hydroxide, calcium oxide, basic aluminium oxide, zinc oxide, and their mixtures. The patch may comprise backing that is a non-permeable to aqueous media. The matrix substantially consists of a material selected from cellulose, polyamide, polyether, polyurethane, their copolymers and mixtures. Additionally the patch may comprise a gel forming agent, such as one selected from hydroxypropyl cellulose (HPC), hydroxymethylpropyl cellulose (HPMC), cross-linked polyvinylpyrrolidone, and cross-lined polyvinyl alcohol.

According to a further preferred aspect is disclosed the use of the patch of the invention for treating warts, wherein the use comprises wetting the patch by an aqueous medium and applying a face of the wetted patch to a wart. The aqueous medium is preferably selected from water and a mixture of water and an alcohol or a mixture of alcohols. It is preferred for the alcohol to be selected from mono- to trivalent alcohol. The aqueous medium may additionally comprise a gel forming agent, such as one selected from hydroxypropyl cellulose (HPC), hydroxymethylpropyl cellulose (HPMC), cross-linked polyvinylpyrrolidone, and cross-linked polyvinyl alcohol.

The invention will now be described in more detail by reference to a number of preferred but not limiting embodiments.

### DESCRIPTION OF PREFERRED EMBODIMENTS

### Example 1

Medical dithionite patch. The free large face of a bleached soft cotton patch 3 x 3 x 0.5 cm in size comprising a polyethylene backing is sprayed with 1 mL of aqueous sodium triphosphate (2 %, w/v). After drying the same face of the patch is sprayed in a nitrogen atmosphere with 1 mL of 5 % (w/v) aqueous sodium dithionite (technical grade, ca. 85 % w/w, Sigma-Aldrich) and dried under reduced pressure. The patch is packed in a polyethylene bag of about same size lined with aluminium foil, and sealed.

### Example 2

*Treatment of warts I.* For treatment the patch of Example 1 is extracted from the bag and 1 ml of water is pipetted in a centred circle about 2 mm in diameter on the free large face of the patch. The patch is immediately pressed with its free face on a skin portion comprising a wart in a centred manner and kept in this position by adhesive strips. After about one hour the patch is taken off. The softened wart can be removed by use of a sterilized wooden spatula. It may be appropriate to wait for some time after removal of the patch before removing the softened wart.

### Example 3

*Treatment of warts II*. Several drops of freshly prepared aqueous solution of sodium dithionite containing 2.0 % by weight of hydroxypropylmethyl cellulose are applied to a common wart on the back of a persons hand. After half an hour the hand is rinsed with water. Three hours from application the wart is wetted. It is now easily removed by means of a wooden spatula.

### Example 4

*Epithelial cell assay.* In an epithelial cytotoxicity cell assay (Meth Cell Sci 22, 17-24, 2000) on AIM V® medium (Invitrogen) at concentrations of 0.001 g/mL, 0.01 g/mL and 0.05 g/mL of sodium dithionite (85 %, Sigma-Aldrich) the lowest concentration did not affect the cells whereas the highest concentration of 0.5% resulted in substantial cell death.

## Claims

1. A pharmaceutical composition for use in the treatment of common warts (*Verruca vulgaris*) wherein the composition is for topical administration and comprises any of sodium, potassium, magnesium, calcium and zinc dithionite and their mixtures in a pharmaceutically acceptable aqueous carrier selected from water or a mixture of water and non-reducible organic solvent selected from ethanol, ethylene glycol, glycerol, and their mixtures, and wherein the dithionite from which the aqueous solution has been prepared is stabilized by any of sodium hydroxide, potassium hydroxide, sodium carbonate, potassium carbonate, magnesium hydroxide, magnesium oxide, magnesium carbonate, basic magnesium carbonate, calcium hydroxide, calcium oxide, basic aluminium oxide, zinc oxide, trisodium phosphate, sodium and potassium salts of organic acids, such as sodium acetate, disodium maleate and trisodium citrate, and their mixtures, with the proviso that the pharmaceutical composition does not comprise an organo-silicon compound.

2. The composition for use according to claim 1, wherein the composition comprises a non-reducible gelling agent, wherein the non-reducible gelling agent comprises a cellulose derivative or a mixture of cellulose derivatives.

3. The composition for use according to claim 2, wherein the non-reducible gelling agent is selected from hydroxypropyl cellulose, (HPC) and hydroxymethylpropyl cellulose (HPMC), cross-linked polyvinylpyrrolidone, and cross-linked polyvinyl alcohol.

4. A medical patch comprising solid dithionite in a nonreducible matrix for application to common warts (*Verruca vulgaris*) upon wetting the patch by an aqueous medium, wherein the matrix substantially consists of a material selected from cellulose, polyamide, polyether, polyurethane, their copolymers and mixtures, and with the proviso that the patch does not comprise an organo-silicon compound.

5. The patch of claim 4, wherein the dithionite is selected from sodium, potassium, magnesium, calcium and zinc dithionite, and their mixtures.

6. The patch of claim 4 or 5, wherein the dithionite is stabilized by any of sodium hydroxide, potassium hydroxide, sodium carbonate, potassium carbonate, magnesium hydroxide, magnesium oxide, magnesium carbonate, basic magnesium carbonate, calcium hydroxide, calcium oxide, basic aluminium oxide, zinc oxide, and their mixtures.

7. The patch of any of claims 4 to 6, comprising a non-permeable backing.

8. The patch of any of claims 4 to 7, comprising a gel forming agent.

9. The patch of claim 8, wherein the gel forming agent is selected from hydroxypropyl cellulose (HPC), hydroxymethylpropyl cellulose (HPMC), cross-linked polyvinylpyrrolidone, and cross-linked polyvinyl alcohol.

10. Dithionite for use in the treatment of common warts (*Verruca vulgaris*), wherein the dithionite is comprised by a patch of any of claims 4-9 wetted by an aqueous medium selected from water, a mixture of water and alcohol or a mixture of alcohols.

11. A pharmaceutical composition for topical administration comprising any of sodium, potassium, magnesium, calcium and zinc dithionite and their mixtures in a pharmaceutically acceptable aqueous carrier selected from water or a mixture of water and non-reducible organic solvent selected from ethanol, ethylene glycol, glycerol, and their mixtures, and wherein the dithionite from which the aqueous solution has been prepared is stabilized by any of sodium hydroxide, potassium hydroxide, sodium carbonate, potassium carbonate, magnesium hydroxide, magnesium oxide, magnesium carbonate, basic magnesium carbonate, calcium hydroxide, calcium oxide, basic aluminium oxide, zinc oxide, trisodium phosphate, sodium and potassium salts of organic acids, such as sodium acetate, disodium maleate and trisodium citrate, and their mixtures, and wherein the composition comprises a non-reducible gelling agent comprising a cellulose derivative or a mixture of cellulose derivatives, with the proviso that the pharmaceutical composition does not comprise an organo-silicon compound.

12. A composition according to claim 11, wherein the non-reducible gelling agent is selected from hydroxypropyl cellulose, (HPC) and hydroxymethylpropyl cellulose (HPMC), cross-linked polyvinylpyrrolidone, and cross-linked polyvinyl alcohol.

## Patentansprüche

1. Pharmazeutische Zusammensetzung zur Verwendung bei der Behandlung von Stachelwarzen (*Verruca vulgaris*), wobei die Zusammensetzung für die topische Verabreichung vorgesehen ist und Natriumdithionit, Kaliumdithionit, Magnesiumdithionit, Calciumdithionit oder Zinkdithionit oder deren Gemische in einem pharmazeutisch akzeptablen wässrigen Träger enthält, der unter Wasser oder einem Gemisch aus Wasser und einem nicht reduzierbaren organischen Lösemittel ausgewählt ist, das unter Ethanol, Ethylenglycol, Glycerin und deren Gemischen ausgewählt ist, wobei das Dithionit, mit dem die wässrige Lösung hergestellt wurde, mit Natriumhydroxid, Kaliumhydroxid, Natriumcarbonat, Kaliumcarbonat, Magnesiumhydroxid, Magnesiumoxid, Magnesiumcarbonat, basischem Magnesiumcarbonat, Calciumhydroxid, Calciumoxid, basischem Aluminiumoxid, Zinkoxid, Trinatriumphosphat, Natrium- und Kaliumsalzen von organischen Säuren, wie Natriumacetat, Dinatriummaleat und Trinatriumcitrat, oder deren Gemischen stabilisiert ist, mit der Maßgabe, dass die pharmazeutische Zusammensetzung keine Silicium-organische Verbindung enthält.

2. Zusammensetzung zur Verwendung nach Anspruch 1, wobei die Zusammensetzung einen nicht reduzierbaren Gelbildner enthält, wobei der nicht reduzierbare Gelbildner ein Cellulosederivat oder ein Gemisch von Cellulosederivaten umfasst.

3. Zusammensetzung zur Verwendung nach Anspruch 1, wobei der nicht reduzierbare Gelbildner unter Hydroxypropylcellulose (HPC) und Hydroxymethylpropylcellulose (HPMC), vernetztem Polyvinylpyrrolidon und vernetztem Polyvinylalkohol ausgewählt ist.

4. Medizinisches Pflaster, das ein festes Dithionit in einer nicht reduzierbaren Matrix enthält, für die Anwendung bei Stachelwarzen (*Verruca vulgaris*) unter Benetzen des Pflasters mit einem wässrigen Medium, wobei die Matrix im Wesentlichen aus einem Material besteht, das unter Cellulose, Polyamid, Polyether, Polyurethan, deren Copolymeren und deren Gemischen ausgewählt ist, mit der Maßgabe, dass das Pflaster keine Silicium-organische Verbindung enthält.

5. Pflaster nach Anspruch 4, wobei das Dithionit unter Natriumdithionit, Kaliumdithionit, Magnesiumdithionit, Calciumdithionit und Zinkdithionit und deren Gemischen ausgewählt ist.

6. Pflaster nach Anspruch 4 oder Anspruch 5, wobei das Dithionitmit Natriumhydroxid, Kaliumhydroxid, Natriumcarbonat, Kaliumcarbonat, Magnesiumhydroxid, Magnesiumoxid, Magnesiumcarbonat, basischem Magnesiumcarbonat, Calciumhydroxid, Calciumoxid, basischem Aluminiumoxid, Zinkoxid und deren Gemischen stabilisiert ist.

7. Pflaster nach einem der Ansprüche 4 bis 6, das eine nicht permeable Rückseite aufweist.

8. Pflaster nach einem der Ansprüche 4 bis 7, das einen Gelbildner umfasst.

9. Pflaster nach Anspruch 8, wobei der Gelbildner unter Hydroxy-propylcellulose (HPC) und Hydroxymethylpropylcellulose (HPMC), vernetztem Polyvinylpyrrolidon und vernetztem Polyvinylalkohol ausgewählt ist.

10. Dithionit zur Verwendung bei der Behandlung von Stachelwarzen (*Verruca vulgaris*), wobei das Dithionit in einem Pflaster nach einem der Ansprüche 4 bis 9 enthalten ist, das mit einem wässrigen Medium angefeuchtet ist, das unter Wasser, einem Gemisch aus Wasser und einem Alkohol oder einem Gemisch von Alkoholen ausgewählt ist.

11. Pharmazeutische Zusammensetzung für die topische Anwendung, die Natriumdithionit, Kaliumdithionit, Magnesiumdithionit, Calciumdithionit oder Zinkdithionit oder deren Gemische in einem pharmazeutisch akzeptablen wässrigen Träger enthält, der unter Wasser oder einem Gemisch aus Wasser und einem nicht reduzierbaren organischen Lösemittel ausgewählt ist, das unter Ethanol, Ethylenglycol, Glycerin und deren Gemischen ausgewählt ist, wobei das Dithionit, mit dem die wässrige Lösung hergestellt wurde, mit Natriumhydroxid, Kaliumhydroxid, Natriumcarbonat, Kaliumcarbonat, Magnesiumhydroxid, Magnesiumoxid, Magnesiumcarbonat, basischem Magnesiumcarbonat, Calciumhydroxid, Calciumoxid, basischem Aluminiumoxid, Zinkoxid, Trinatriumphosphat, Natrium- und Kaliumsalzen von organischen Säuren, wie Natriumacetat, Dinatriummaleat und Trinatriumcitrat, oder deren Gemischen stabilisiert ist, und wobei die Zusammensetzung einen nicht reduzierbaren Gelbildner enthält, der unter einem Cellulosederivat oder einem Gemisch von Cellulosederivaten ausgewählt ist, mit der Maßgabe, dass die pharmazeutische Zusammensetzung keine Silicium-organische Verbindung enthält.

12. Zusammensetzung nach Anspruch 11, wobei der nicht reduzierbare Gelbildner unter Hydroxypropylcellulose (HPC) und Hydroxymethylpropylcellulose (HPMC), vernetztem Polyvinylpyrrolidon und vernetztem Polyvinylalkohol ausgewählt ist.

## Revendications

1. Composition pharmaceutique pour une utilisation dans le traitement des verrues vulgaires (*Verruca vulgaris*), ladite composition étant pour l'administration topique et comprenant n'importe lequel des dithionites de sodium, de potassium, de magnésium, de calcium et de zinc et leurs mélanges dans un véhicule aqueux pharmaceutiquement acceptable choisi entre l'eau et un mélange d'eau et d'un solvant organique non réductible choisi entre l'éthanol, l'éthylèneglycol, le glycérol et leurs mélanges, le dithionite à partir duquel la solution aqueuse a été préparée étant stabilisé par n'importe lequel de l'hydroxyde de sodium, de l'hydroxyde de potassium, du carbonate de sodium, du carbonate de potassium, de l'hydroxyde de magnésium, de l'oxyde de magnésium, du carbonate de magnésium, du carbonate basique de magnésium, de l'hydroxyde de calcium, de l'oxyde de calcium, de l'oxyde basique d'aluminium, de l'oxyde de zinc, du phosphate trisodique, des sels de sodium et de potassium d'acides organiques tels que l'acétate de sodium, le maléate disodique et l'acide trisodique, et de leurs mélanges, sous réserve que la composition pharmaceutique ne comprenne pas de composant organique de silicium.

2. Composition pour une utilisation suivant la revendication 1, ladite composition comprenant un agent gélifiant non réductible, dans laquelle l'agent gélifiant non réductible comprend un dérivé de cellulose ou un mélange de dérivés de cellulose.

3. Composition pour une utilisation suivant la revendication 2, dans laquelle l'agent gélifiant non réductible est choisi entre l'hydroxypropylcellulose (HPC) et hydroxyméthylpropylcellulose (HPMC), la polyvinylpyrrolidone réticulée et l'alcool polyvinyle réticulé.

4. Timbre médical comprenant du dithionite de sodium dans une matrice non réductible pour l'application aux verrues vulgaires (*Verruca vulgaris*) par mouillage du timbre par un milieu aqueux, dans lequel la matrice consiste substantiellement en une matière choisie entre la cellulose, un polyamide, un polyéther, un polyuréthanne, leurs copolymères et mélanges, et sous réserve que le timbre ne comprenne pas de composé organique de silicium.

5. Timbre suivant la revendication 4, dans lequel le dithionite est choisi entre les dithionites de sodium, de potassium, de magnésium, de calcium et de zinc et leurs mélanges.

6. Timbre suivant la revendication 4 ou 5, dans lequel le dithionite est stabilisé dans n'importe lequel de l'hydroxyde de sodium, de l'hydroxyde de potassium, du carbonate de sodium, du carbonate de potassium, de l'hydroxyde de magnésium, de l'oxyde de magnésium, du carbonate de magnésium, du carbonate basique de magnésium, de l'hydroxyde de calcium, de l'oxyde de calcium, de l'oxyde basique d'aluminium, de l'oxyde de zinc et de leurs mélanges.

7. Timbre suivant l'une quelconque des revendications 4 à 6, comprenant une couche de support non perméable.

8. Timbre suivant l'une quelconque des revendications 4 à 7, comprenant un agent de formation de gel.

9. Timbre suivant la revendication 8, dans lequel l'agent de formation de gel est choisi entre l'hydroxypropylcellulose (HPC), l'hydroxyméthylpropylcellulose (HPMC), la polyvinylpyrrolidone réticulée et l'alcool polyvinylique réticulé.

10. Dithionite pour une utilisation dans le traitement des verrues vulgaires (*Verruca vulgaris*), ledit dithionite étant constitué d'un timbre de l'une quelconque des revendications 4 à 9 mouillé par un milieu aqueux choisi entre l'eau, un mélange d'eau et d'un alcool et un mélange d'alcools.

11. Composition pharmaceutique pour l'administration topique, comprenant n'importe lequel des dithionites de sodium, de potassium, de magnésium et de zinc et de leurs mélanges dans un véhicule aqueux pharmaceutiquement acceptable choisi entre l'eau et un mélange d'eau et d'un solvant organique non réductible choisi entre l'éthanol, l'éthylèneglycol, le glycérol et leurs mélanges, dans laquelle le dithionite à partir duquel la solution aqueuse a été préparée est stabilisé par n'importe lequel de l'hydroxyde de sodium, de l'hydroxyde de potassium, du carbonate de sodium, du carbonate de potassium, de l'hydroxyde de magnésium, de l'oxyde de magnésium, du carbonate de magnésium, du carbonate basique de magnésium, de l'hydroxyde de calcium, de l'oxyde de calcium, de l'oxyde basique d'aluminium, de l'oxyde de zinc, du phosphate trisodique, des sels de sodium et de potassium, d'acides organiques tels que l'acétate de sodium, le maléate disodique et l'acide trisodique, et de leurs mélanges, ladite composition comprenant un agent gélifiant non réductible comprenant un dérivé de cellulose ou un mélange de dérivés de cellulose, sous réserve que la composition pharmaceutique ne comprenne pas de composé organique de silicium.

12. Composition suivant la revendication 11, dans laquelle l'agent gélifiant non réductible est choisi entre l'hydroxypropylcellulose (HPC) et l'hydroxyméthylpropylcellulose (HPMC), la polyvinylpyrrolidone réticulée et l'alcool polyvinylique réticulé.
